# EUROPÄISCHE PATENTSCHRIFT

(11) **EP 0 358 130 B1**
(45) Veröffentlichungstag und Bekanntmachung des Hinweises auf die Patenterteilung: **11.08.1993**
(21) Anmeldenummer: 89116210.9
(22) Anmeldetag: 01.09.1989
(51) Int. Cl.: A61K 37/02, C07K 15/06, C12P 21/00, A61K 39/395

(54) **Mittel mit immunsuppressiver Wirkung**
Agent with an immunosuppressive activity
Remède possédant un effet immunosupressif

(30) Priorität: 06.09.1988 DE 3830271
(43) Veröffentlichungstag der Anmeldung: 14.03.1990
(73) Patentinhaber: Götze, Otto Prof. Dr. med., D-37075 Göttingen (DE)
(72) Erfinder: Götze, Otto Prof. Dr. med., D-37075 Göttingen (DE)
(74) Vertreter: Grünecker, Kinkeldey, Stockmair & Schwanhäusser Anwaltssozietät

(56) Entgegenhaltungen:
- IMMUNOLOGY TODAY, Band 8, Nr. 9, 1987, Seiten 261-264; E.C. BÖTTGER et al.: "Complement and the regulation of humoral immune responses"
- IMMUNOLOGY LETTERS, Band 9, 1985, Seiten 207-213; E.L. MORGAN et al.: "Bioactive complement fragments in immunoregulation"
- THE JOURNAL OF IMMUNOLOGY, Band 131, Nr. 5, November 1983, Seiten 2258-2261; E.L. MORGAN et al.: "Suppression of humoral immune responses by synthetic C3a peptides"
- THE JOURNAL OF IMMUNOLOGY, Band 134, Nr. 1, Januar 1985, Seiten 51-57; E.L. MORGAN et al.: "I. Suppression of murine in Vitro antobody responses occurs through the generation of nonspecific lyt-2+ suppressor T cell"
- THE JOURNAL OF IMMUNOLOGY, Band 133, Nr. 5, November 1984, Seiten 2629-2633; M.L. THOMAN et al.: "C3d-K, A kallikrein cleavage fragment of iC3b is a potent inhibitor of cellular proliferation"
- J. CLIN PATHOL (ENGLAND), Band 40, Nr. 10, 1987, Seiten 1235-1239; G. SENALDI et al.: "Activation of the alternative complement pathway: clinical application of a new technique to measure fragment Ba"
- TRANSPLANTATION, Band 39, Nr. 5, 1985, Seiten 510-514; W.J. VAN SON et al.: "Complement activation associated with ctive cytomegalovirus infection in renal transplant patients, and its absence in transplant rejection episodes"
- ACTA PATH. MICROBIOL. IMMUNOL. SCAND. Sect. C. Band 92, 1984, Seiten 213-220; J. SOLLING: "Circulating immune complexes and complement breakdown product C3d in glomerulonephritis and kidney transplantation"
- CHEMICAL ABSTRACTS, Band 108, Nr. 11, 1988, Seite 509, Zusammenfassung Nr. 92820s, Columbus, Ohio, US; A.L. PETZER et al.: "Structural and functional analysis of CR2/EBV receptor by means of monoclonal antibodies and limited tryptic digestion", & IMMUNOLOGY 1988, 63(1), 47-53
- CHEMICAL ABSTRACTS, Band 104, Nr. 5, 1986, Seite 475, Zusammenfassung Nr. 223276m, Columbus, Ohio, US; R.T. PERRI et al.: "Inhibition of B cell growth factor (BCGF) by monoclonal antibodies directed against the C3d receptor (CR2)"
- CHEMICAL ABSTRACTS, Band 103, Nr. 7, 1985, Seite 441, Zusammenfassung Nr. 52483z, Columbus, Ohio, US; P.E. RAO et al.: "OKB7, a monoclonal antibody that reacts at or near the C3d binding site of human CR2."
- CHEMICAL ABSTRACTS, Band 108, Nr. 11, 1988, Seite 509, Zusammenfassung Nr. 92819y, Columbus, Ohio, US; A.X. DELCAYRE et al.: "gp140, The EBV/C3d receptor (CR2) of human B lymphocytes, is involved in cell-free phosphorylation of pl20, a nuclear ribonucleoprotein", & EUR. J. IMMUNOL. 1987, 17(12), 1827-33

## Beschreibung

Die Erfindung bezieht sich auf ein Mittel mit immunsuppressiver Wirkung, auf einen Antikörper gegen den Komplementrezeptor Typ 2 (CR2), der die Bindung der Komplementpeptide C3d/C3dg blockiert, auf eine diesen Antikörper enthaltende Zusammensetzung und auf die Verwendung der Antikörper.

Das körpereigene Immunsystem ist ein äußerst komplexes System, das normalerweise dem Schutz des Organismus vor körperfremden Substanzen dient; gegen Strukturen des eigenen Organismus, die während der Embryonalzeit in ständigem Kontakt mit dem heranreifenden Immunapparat gestanden haben, besteht dagegen immunbiologische Toleranz. Es gibt jedoch Erkrankungen, bei denen das Prinzip der immunologischen Toleranz gegen autologes Gewebe durchbrochen wird und Antikörper gegen körpereigene Strukturen gebildet werden. Beispiele für solche Erkrankungen sind Myasthenia gravis, Lupus erythematodes, Leuko-, Thrombo- oder Pancytopenien, Rheuma und wahrscheinlich auch Multiple Sklerose. Diese Krankheiten werden bisher durch physikalische, chemische oder biologische Agenzien, die in der Lage sind, die immunologischen Reaktionen zu unterdrücken bzw. abzuschwächen, behandelt.

Auch eine normale Immunantwort kann bespielsweise bei Patienten, denen körperfremde Organe oder Gewebe transplantiert worden sind, höchst unerwünscht sein und der gezielten Unterdrückung bedürfen.

Transplantationspatienten müssen Zeit ihres Lebens mit immunsuppressiven Mitteln behandelt werden, um eine Abstoßung des fremden Organes bzw. Gewebes durch das Immunsystem zu verhindern.

Bisher zur Behandlung von Autoimmunkrankheiten und zur Prophylaxe von Abstoßungsreaktionen verwendete Immunsuppressiva sind beispielsweise ionisierende Strahlen, die eine allgemeine direkte cytotoxische Wirkung haben, jedoch wegen der zu großen Nebenwirkungen einer Ganzkörperbestrahlung nur in Form einer schwierig durchzuführenden extrakorporalen Bestrahlung des Blutes durchgeführt werden können. Weiterhin werden Corticosteroide eingesetzt, deren anti-inflammatorischer sowie Phagocytose-hemmender Effekt zwar bekannt ist, deren immunsuppressive Wirkung sich jedoch beim Menschen bisher nicht im einzelnen hat aufklären lassen. Weitere derzeit verwendete Immunsuppressiva sind Cytostatika, beispielsweise alkylierende Substanzen wie Cyclophosphamid oder Antimetabolite, wie Methotrexat oder Azathioprin. Alle die genannten Immunsuppressiva haben den erheblichen Nachteil, daß sich ihre Wirkung nicht auf die Zellen des Immunsystems beschränkt, sondern daß sie die Zellproliferation und differenzierung unspezifisch unterdrücken oder gar cytotoxisch sind. Die bisher bekannten Medikamente weisen darüber hinaus einige spezifische Nebenwirkungen auf; beispielsweise ist mit einer Cyclophosphamidbehandlung die Gefahr des Auftretens einer hämorrhagischen Cystitis und einer Ovarialinsuffizienz verbunden, mit einer Methotrexatbehandlung die Gefahr einer Leberzirrhose und mit der Verwendung alkylierender Substanzen das Auftreten von Mutationen in Keimdrüsenzellen.

Aus den in vitro-Versuchen von Morgan et al. (The Journal of Immunology, 131, Seiten 2258 bis 2261, 1983) ist bereits bekannt, daß synthetische Oligopeptide, die die carboxyterminale Sequenz des nativen humanen C3a's enthalten, in vitro immunsuppresiv wirksam sind, allerdings mit einer im Vergleich zum nativen Komplementfaktor C3a bereits erheblich erniedrigten biologischen Wirksamkeit.

Aufgabe der Erfindung ist es daher, Mittel zur Verfügung zu stellen, deren Wirkung in einer spezifischen Immunsuppression besteht.

Diese Aufgabe wird erfindungsgemäß durch ein Mittel gelöst, das Peptide mit der biologischen Funktion der Komplementpeptide Ba und/oder C3a und/oder C3dg und/oder C3d enthält.

Diese Aufgabe wird weiterhin erfindungsgemäß durch einen Antikörper gegen den Komplementrezeptor Typ 2 (CR2), der die Bindung der Komplementpeptide C3d/C3dg blockiert, durch einen monovalenten Antikörper, sowie durch diese Antikörper enthaltende Zusammensetzungen gelöst.

Das erfindungsgemäße Mittel hat den großen Vorteil, daß es ausschließlich aus körpereigenen, im Verlauf der normalen Komplementaktivierung natürlicherweise auftretenden Proteinen besteht, die eine spezifische Wechselwirkung mit den Lymphocyten eingehen und daher keinerlei unspezifische Nebenreaktionen aufweisen. Im Verlauf der Komplementaktivierung wird das Plasmaprotein C3 durch proteolytische Aktivierung in die Komplementpeptide C3a und C3b gespalten. C3b wird durch Spaltung einiger Peptidbindungen in der α-Kette weiter in iC3b verwandelt. iC3b wird dann weiter degradiert in C3dg, ein Peptid aus der α-Kette mit einem Molekulargewicht von 39 kD und C3c. C3dg kann weiter zu C3d und C3g fragmentiert werden. Diese letzte beobachtete Fragmentierung beruht möglicherweise jedoch nur auf der Kontamination bakterieller Proteasen.

Es ist bereits bekannt, daß in Patientengruppen mit pathophysiologisch verminderter Reaktivität des Immunsystems erhöhte Blutkonzentrationen von Ba und aktiviertem C3, einschließlich des Faktors C3dg/C3d, vorliegen. Bei solchen Patienten wurden beispielsweise C3a Plasmaspiegel von 60 ng/ml beobachtet, C3dg Plasmaspiegel von 80 ug/ml und Ba Plasmaspiegel von 30 ug/ml. Die bei diesen Patienten gefundenen erhöhten Blutspiegel der Komplementpeptide zeigen, daß diese Wirkstoffe ohne gravierende Nebenwirkungen in vivo in den beobachteten Konzentrationen vertragen werden. Bei einer therapeutischen Anwendung der Peptide treten daher ausschließlich die gewünschten immunsuppressiven Wirkungen und keinerlei Nebenwirkungen auf. Das erfindungsgemäße Mittel eignet sich dabei insbesondere zur Behandlung von allergischen, atopischen und immunpathologischen Autoaggressionskrankheiten und anderen Erkrankungen, die mit einer erhöhten Aktivität von B-Lymphocyten, von T-Lymphocyten oder von Monocyten und Makrophagen einhergehen, sowie solchen des rheumatischen Formenkreises.

Die verschiedenen Peptide mit der biologischen Funktion mindestens eines der Komplementpeptide können einzeln oder in beliebiger Kombination zugesetzt werden. Nach bisherigen Erkenntnissen aus in vitro-Versuchen wirken sie über verschiedene Mechanismen immunsuppressiv, so daß die Gabe einer Kombination der verschiedenen Peptide zu einem Multiplikationseffekt führt. So ist festgestellt worden, daß Ba die Proliferation der B-Lymphocyten und die Produktion der Immunglobuline IgG und IgM unterdrückt. Offenbar trägt ein kleinerer Teil der aus dem Blut gewonnenen mononukleären Leukozyten, möglicherweise ein Teil der Monozyten oder der Blastzellen, die bereits voraktiviert sind, eine Bindungsstelle für Ba. Die Bindung von Ba in einen solchen zellulären Rezeptor leitet wahrscheinlich die inhibierenden Reaktionen ein.

Für das Peptid C3a hingegen ist eine Suppression der humanen polyklonalen Antikörperproduktion, ebenfalls in vitro, beschrieben worden. Die Wirkung soll über die Aktivierung von T8-positiven Suppressor-T-Zellen erfolgen. Adhärente Zellen, wahrscheinlich Monocyten bzw. Macrophagen, sind für die Ausbildung der suppressorischen Aktivität notwendig. Für das Peptid C3dg/C3d ist ebenfalls in vitro unter Verwendung von Leucocyten der Maus eine Inhibition der Proliferation von B-Lymphocyten gezeigt worden. Dabei unterdrückte lösliches C3d in Konzentrationen von mehr als 2 ug/ml die durch Antikörper gegen das Oberflächenimmunglobulin der B-Lymphocyten in Gegenwart anderer löslicher Faktoren aus Macrophagen ausgelöste Zellteilung der B-Lymphocyten. Aggregiertes C3dg/C3d führt dagegen zu einer Stimulation der Zellproliferation. Es ist daher wichtig, im erfindungsgemäßen Mittel monomeres C3dg/C3d zur Verfügung zu stellen, in dem beispielsweise die Bildung von Disulfidbrücken durch Zugabe physiologisch verträglicher Agenzien, wie Cystein, oder durch andere Methoden der Blockade von SH-Gruppen, verhindert wird.

Die beobachteten Effekte werden nicht nur durch die vollständigen Peptide hervorgerufen, sondern können auch durch den jeweils für die Funktion verantwortlichen Teil des entsprechenden Peptides verursacht werden. So ist bereits bekannt, daß ein synthetisches Peptid, das den Carboxy-terminalen zehn Aminosäuren des humanen Ba entspricht, die Proliferation der B-Zellen merklich inhibiert (J.L. Ambrus, Jr., M.G. Peters, A.S. Fauci und E.J. Brown, Fed. Proc. 46, 1216, Abstract 5241, 1987). Bereits dieses Beispiel macht deutlich, daß Teilpeptide, die nach wie vor die biologische Funktion des jeweiligen Komplementpeptides erfüllen, als Bestandteil des Mittels genauso geeignet sind, sofern sie noch die biologische Funktion des aktiven Komplementfragmentes erfüllen.

Erfindungsgemäß können die Peptide durch proteolytische Spaltung natürlicher, aus Spenderblut isolierter Komplementfaktoren erhalten werden. Beispiele für die Isolierung solcher Faktoren finden sich beispielsweise in folgenden Publikationen: Gewinnung von Ba: O. Götze, H.J. Müller-Eberhard, J. Exp. Med. 134, 90s (1971); Gewinnung von C3a: T.E. Hugli, E.M. Vallota, H.J. Müller-Eberhard, J. Biol. Chem. 250, 1472 (1975) und M. Oppermann, F. Liebmann, O. Götze, Complement 4, 205 (1987); Gewinnung von C3dg bzw. C3d in Anlehnung an D.P. Vick, D.T. Fearon, J. Immunol. 134, 2571 (1988). Nachdem die Aminosäuresequenzen der in Frage stehenden Komplementfaktoren und die Nukleotidsequenz des Komplementproteins C3 inzwischen bekannt sind (M.H.L. de Bruijn, G.H. Fey, Proc. Natl. Acad. Sci. USA 82, 708-712, 1985), können die Peptide auch durch biologische Synthese gewonnen werden. Das bedeutet, daß beispielsweise entweder die natürlichen Gene dieser Komplementfaktoren oder in vitro in Übereinstimmung mit den bekannten Aminosäuresequenzen synthetisierte DNA-Doppelstränge in Expressionsvektoren kloniert werden können, die die effiziente Expression der gewünschten Komplementfaktoren in einem geeigneten Wirtsorganismus bewirken. Als Wirtsorganismen kommen beispielsweise Escherichia coli oder Hefe in Betracht. Die dafür erforderlichen Methoden sind dem Fachmann spätestens seit der biologischen Synthese des Insulins geläufig. Weiterhin ist eine totale chemische Synthese der Peptide mit der biologischen Funktion der genannten Komplementpeptide möglich. Ein dafür geeignetes Verfahren ist beispielsweise die Merrifield-Synthese. Unter bestimmten Bedingungen kann sich auch eine kombinierte biologisch/chemische Synthese als nützlich erweisen, beispielsweise bei der chemischen Spaltung oder Modifizierung von biologisch synthetisierten Vorläufern.

Das erfindungsgemäße Mittel wird beispielsweise parenteral systemisch, lokal oder oral in Form eines Aerosols verwendet. Dazu werden die Peptide mit der biologischen Funktion der Komplementpeptide mit üblichen Zusatzmitteln, Verdünnungsmitteln und/oder Hilfsmitteln versetzt, um eine für die gewünschte Applikationsform geeignete Zubereitung zu erhalten.

Vorzugsweise wird das erfindungsgemäße Mittel als wässrige Lösung infundiert, oder als Wasser-in-Öl Emulsion oder in Liposomen inkorporiert lokal appliziert. Weiter mögliche Verwendungsformen sind die Inhalation eines Aerosols, beispielsweise bei allergischen Asthmaanfällen, oder eine Konfektionierung für die orale Einnahme.

Eine bevorzugte Ausführungsform der vorliegenden Erfindung sieht ein Mittel vor, das neben den Peptiden mit der biologischen Funktion eines oder mehrerer der Komplementpeptide weitere immunsuppressive Wirkstoffe enthält. Dabei kann es sich beispielsweise um Cytostatika oder Corticosteroide handeln. Durch die gleichzeitige Verwendung dieser bereits bekannten immunsuppressiven Agenzien und der Komplementpeptide läßt sich ein überraschend hoher immunsuppressiver Effekt bei Verwendung sehr niedriger Konzentrationen des vom Komplementpeptid verschiedenen immunsuppressiven Wirkstoffes erzielen. Dadurch verringern sich die unerwünschten Nebenwirkungen der früher ausschließlich verwendeten Immunsuppressiva.

Die oben genannte Aufgabe, nämlich, Mittel zur Verfügung zu stellen, deren Wirkung in einer spezifischen Immunsuppression besteht, wird weiterhin überraschenderweise durch einen Antikörper gegen den Komplementrezeptor Typ 2 (CR2) gelöst, der die Bindung der Komplementpeptide C3dg/C3d blockiert und die Aggregation von Rezeptormolekülen inhibiert, sowie durch einen monovalenten Antikörper mit der gleichen Funktion und durch diese Antikörper enthaltende Zusammensetzungen.

Es ist bereits bekannt, daß durch Bindung von polymerem C3dg/C3d an den Rezeptor CR2 der Eintritt aktivierter B-Zellen in die S-Phase kontrolliert wird (F. Melchers, A. Erdei, Th. Schulz, M.P. Dierich, Nature 317, 264-267, 1985), also die B-Zellproliferation stimuliert wird. Andererseits scheint monomeres C3dg/C3d den entgegengesetzten Effekt hervorzurufen, wahrscheinlich, indem es die Bindung polymerer Peptide kompetitiv inhibiert. Melchers et al. (s.o.) erklären sich die inhibierende Wirkung monomeren C3d's damit, daß die Besetzung der Rezeptoren durch den monomeren Liganden eine Rezeptoraggregation in der Ebene der Membran durch die Einwirkung polymeren C3dg's/C3d's nicht mehr zuläßt. Diese Rezeptoraggregation ist aber wahrscheinlich einer der physiologischen Mechanismen der Aktivierung der B-Lymphocyten durch das Komplementsystem. Ist diese Aggregation durch Besetzung der Rezeptoren mit monomeren Liganden blockiert, können die Zellen wahrscheinlich nicht mehr effizient stimuliert werden. Diesen inhibierenden Effekt kann ein Antikörper gegen den Rezeptor CR2 imitieren, indem er durch Besetzen der Komplementpeptid-bindenden Rezeptoren mit Antikörpern die Bindung der Komplementpeptide C3dg/C3d blockiert und die durch die Dimerisierung oder Polymerisierung dieser Peptide hervorgerufene Aggregation der Rezeptoren und damit die Stimulation der Immunantwort verhindert. Die Inhibition der Immunantwort durch einen spezifisch mit dem Rezeptor CR2 reagierenden Antikörper hat den wesentlichen Vorteil, daß ein solcher Antikörper mit sehr viel weniger Aufwand in sehr viel größeren Mengen und größerer Reinheit herstellbar ist, als das entsprechende monomere Komplementpeptid C3dg/C3d, dessen Polymerisation durch geeignete Stabilisatoren der monomeren Form überdies verhindert werden müßte. In einer bevorzugten Ausführungsform werden dabei monoklonale Antikörper verwendet. Der Vorteil einer solchen Präparation besteht darin, daß sie ausschließlich Antikörper einer einzigen, definierten Spezifität enthält. Weiterhin handelt es sich bei monoklonalen Antikörpern um in vitro produzierte Proteine, die in der Regel die Gefahr von Verunreinigungen beispielsweise mit pathogenen Viren oder ähnlichem ausschließen. Bei den bisher bekannten monoklonalen Antikörpern handelt es sich um Maus-Immunglobuline. Die Herstellung humaner monoklonaler Antikörper ist jedoch ebenso möglich.

Eine weitere bevorzugte Ausführungsform sieht die Bereitstellung von monovalenten Antikörperfragmenten vor. Solche Fragmente werden in an sich bekannter Weise durch Spaltung bivalenter Antikörper hergestellt. Monovalente Fragmente sind z.B. Fab-Fragmente, die durch Spaltung mit Papain gewonnen werden, oder Fab′-Fragmente, die durch Pepsinspaltung und Reduktion des dabei entstehenden F(ab′)₂-Fragmentes erzeugt werden. Diese Fragmente sind nicht in der Lage, auch nur eine Dimerisierung von Rezeptormolekülen herbeizuführen, und eignen sich daher ganz besonders für eine effektive Inhibition der Rezeptoraggregation. Bevorzugt wird für die Herstellung solcher monovalenter Antikörper ein monoklonaler Antikörper gegen den CR2 Rezeptor verwendet.

Da die Nukleotidsequenz des humanen CR2-Proteins bekannt ist (J.J. Weis, L.E. Toothacker, S.R. Burrow, J.H. Weis, D.T. Fearon, Complement 4, 238, 1987), können monoklonale Antikörper gegen diesen Rezeptor nach bekannten Verfahren hergestellt werden. Dafür werden beispielsweise in vivo exponierte antigene Bereiche dieses Proteines festgestellt und anschließend synthetische Peptide, die diesen antigenen Bereichen entsprechen, synthetisiert und an einen geeigneten Träger gekoppelt, um damit beispielsweise Mäuse zu immunisieren. Die weitere Herstellung und Selektion von Hybridzell-Linien, die monoklonale Antikörper gegen CR2-Protein herstellen, erfolgt nach dem Fachmann bekannten Verfahren analog dem erstmals von Köhler und Milstein (Nature 256, 495-497, 1975) beschriebenen Verfahren.

Zur Applikation als immunsuppressiver Wirkstoff wird dieser Antikörper in Form einer Zusammensetzung zur Verfügung gestellt. Die erfindungsgemäße Zusammensetzung kann dabei sowohl die intakten Immunglobuline enthalten, die aus Zellkulturen gewonnen werden, oder Antikörperfragmente, die durch enzymatische Spaltung des Antikörpers erhalten werden. Beispiele solcher Fragmente sind z.B. die Fab-, Fab′-, F(ab)₂- oder F(ab′)₂-Fragmente. Bevorzugt sind insbesondere monovalente Antikörperfragmente, weil durch sie die Aggregation von auch nur 2 Rezeptormolekülen nicht beschleunigt wird. Darüber hinaus ist es möglich, die Antikörperfragmente bis auf den eigentlich für die Wechselwirkung mit dem Rezeptor CR2 verantwortlichen Bereiche zu verkleinern. Ein solches Peptid kann dann entweder durch gezielte Proteolyse der Antikörper oder aber durch chemische Synthese bereitgestellt werden.

Auch im Zusammenhang mit der Verwendung von Zusammensetzungen, die Antikörper gegen den CR2-Rezeptor, bzw. Fragmente dieser Antikörper enthalten, hat es sich gezeigt, daß ein Kombinationspräparat, das neben dem Antikörper oder seinen Fragmenten bekannten chemische Immunsuppressiva enthält, eine überraschend große Wirkung entfaltet. Die Kombination dieser beiden Klassen von Wirkstoffen ermöglicht eine starke Reduktion der notwendigen Dosis chemischer Immunsuppressiva.

Die erfindungsgemäße Zusammensetzung kann weiterhin übliche Verdünnungs- und Zusatzmittel enthalten, beispielsweise Puffersubstanzen, Salze, insbesondere Natriumchlorid zur Einstellung eine iso-osmolaren Lösung, Stabilisatoren und weitere übliche Hilfsmittel.

Eine weitere bevorzugte Ausführungsform des erfindungsgemäßen Mittels besteht in einer Kombination eines Mittels, das eines oder mehrere der Komplementpeptide mit der biologischen Funktion der Peptide Ba und/oder C3a und/oder C3dg und/oder C3d sowie gegebenenfalls weitere immunsuppressive Wirkstoffe enthält, und das darüber hinaus einen Antikörper, der spezifisch mit dem Rezeptor CR2 reagiert, oder Fragmente davon beinhaltet. Ein solches Mittel entfaltet auf allen zur Zeit denkbaren Ebenen eine immunsuppressive Wirkung.

Das erfindungsgemäße Mittel kann erfolgreich zur Immunsuppression bei Säugetieren, insbesondere beim Menschen verwendet werden.

Erfindungsgemäß wird eine Zusammensetzung, die den monoklonalen Antikörper oder Fragmente davon enthält, ebenfalls zur Immunsuppression eingesetzt. Ihre Wirkung beruht auf der Inhibition der Aggregation der Rezeptormoleküle CR2.

Bevorzugte Anwendungsgebiete für die erfindungsgemäßen Mittel und Zusammensetzungen sind Immunkomplexerkrankungen, beispielsweise Myasthenia gravis, Lupus erythematodes, Leuko-, Thrombo- oder Pancytopenien, rheumatische Polyarthritis und andere Erkrankungen des rheumatischen Formenkreises sowie Multiple Sklerose, außerdem die Therapie und Prophylaxe von Abstoßungsreaktionen bei Transplantationspatienten.

Die Figuren und Beispiele erläutern die Erfindung.

### Figurenbeschreibung

Figur 1: Einfluß des Komplementpeptides Ba auf die Gesamt-Immunglobulinsynthese bzw. -freisetzung in vitro. Der der Figur zugrundeliegende Versuch ist in Beispiel 2.1 beschrieben.

Figur 2: Einfluß von Ba auf die IgG- und IgM-Synthese in vitro. Der entsprechende Versuch ist in Beispiel 2.2 beschrieben. Dreiecke symbolisieren für IgM gemessene Werte, während offene Kreise die jeweiligen IgG-Konzentrationen bedeuten.

Figur 3: Einfluß des Komplementpeptides Ba auf die IgG- bzw. IgM-Synthese in LPS-stimulierten Zellkulturen. Der entsprechende Versuch ist in Beispiel 2.3 beschrieben.

Figur 4: Vergleich des Einflusses des Komplementproteines B mit dem des Komplementpeptides Ba auf die Proliferation von mononukleären Blutleukozyten.

### Beispiel 1

Die Komplementpeptide Ba, C3a, C3dg und C3d werden nach dem oben erwähnten Verfahren aus dem Serum von Spenderblut isoliert. Die isolierten Peptide werden in Mengen von 20 bis 100 mg Peptid in physiologischer, phosphatgepufferter Kochsalzlösung bei pH 7,0 +/- 0,5 einzeln oder in Kombination verschiedener Peptide gelöst.

Die Applikation der Komplementpeptide erfolgt bevorzugt über eine Infusionsbehandlung. Dabei werden den Patienten täglich 0,5 bis 1 mg eines oder mehrerer der erwähnten Komplementpeptide pro Kilogramm Körpergewicht in einem Gesamtvolumen zwischen 50 und 200 ml der folgenden Lösung infundiert.

| | |
|---|---|
| Natriumdihydrogenphosphat | 0,45 g |
| Dinatriumhydrogenphosphat | 1,8 g |
| Natriumchlorid | 8,6 g |
| Polysorbat 80^{R} | 0,2 g |
| Wasser | ad 1 l pH 7,0 +/- 0,5 |

### Beispiel 2

### Inhibition der Immunglobulinsynthese bzw. -freisetzung durch Komplementpeptid Ba

### 2.1: Einfluß von Ba auf die Gesamt-Immunglobulinsynthese in vitro.

Ba wurde nach der Methode von O. Götze und H.J. Müller-Eberhard, J.Exp.Med. 134, 90s (1971), hergestellt. In dieser Publikation wird Ba mit b-Fragment bezeichnet. Das erhaltene Ba-Fragment (Fig. 5 der Publikation) wurde wie folgt weiter aufgereinigt. Zuerst wurde eine Gelfiltration auf Sephadex G75SF (Pharmacia, Freiburg) in 30 mM Phosphat, 250 mM NaCl, pH 7,2 durchgeführt. Anschließend wurden die Ba-haltigen Fraktionen (Molekulargewicht etwa 33.000) auf Mono-Q (Pharmacia, Freiburg) weiter gereinigt. Diese Reinigung erfolgte in 20 mM TRIS-HCl, pH 7,6, mit einem Gradienten von 0-300 mM NaCl. Ba eluiert bei etwa 150 mM NaCl von der Säule.

Mononukleäre Blutleukozyten von drei gesunden Spendern wurden auf eine Konzentration von 1,5 x 10⁶ Zellen/ml Serum-freies Iscove's Medium eingestellt. Je 1 ml dieser Suspension wurde 7 Tage bei 37°C im Inkubator (Inkubationsbedingungen: 5 % CO₂, 95 % Luft) mit unterschiedlichen Konzentrationen des wie oben beschriebenen isolierten Ba inkubiert. Der Versuch wurde mit 0, 0,045, 0,09, 0,18, 0,375, 0,75 und 1,5 uM Ba durchgeführt.

Nach 7 Tagen wurde jeweils ein Aliquot von 0,1 ml des Überstandes von insgesamt 1 ml der Zellkulturen abgenommen und die gebildeten Immunglobuline mittels ELISA-Verfahren quantifiziert. Das ELISA-Verfahren wurde wie folgt durchgeführt.

Ein erster Antikörper (anti-Human Ig von der Ziege, affinitätsgereinigt von Dianova, Hamburg) wurde in die Mikrotiterplatte geschichtet. Nach Inkubation mit den von den mononuklearen Blutleukozyten gebildeten Immunglobulinen wurden diese durch Reaktion mit den gleichen Antikörpern wie zuvor, aber in Biotinkonjugierter Form (Dianova, Hamburg), quantifiziert. Die Bindung des zweiten Antikörpers wurde mit einem 1:1000 verdünnten Streptavidin-Peroxidasekonjugat (Amersham, Braunschweig) nachgewiesen.

Die Volumina aller pipettierten Proben und Reagenzien betrugen 0,1 ml. Die Konzentrationen bzw. die Verdünnungen der Reagenzien sind für eine lineare Nachweiskurve optimiert worden.

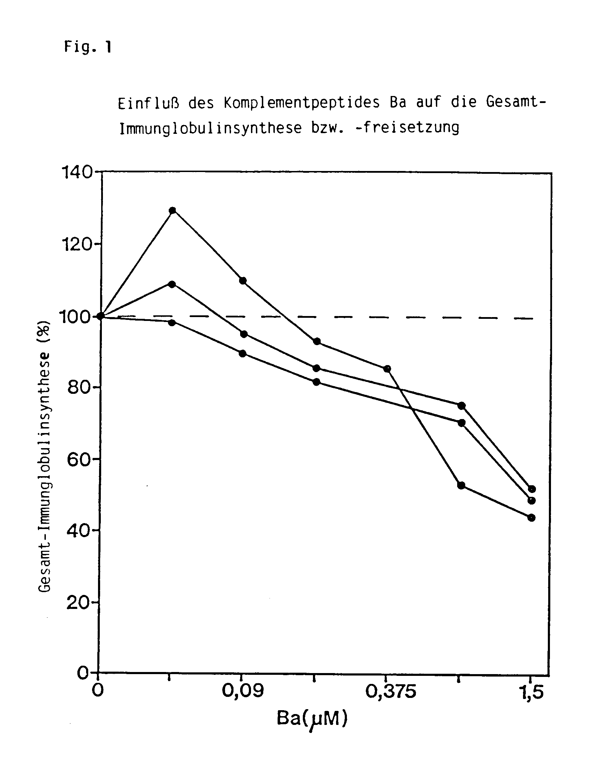

Das Ergebnis des Versuches ist in Figur 1 dargestellt. Aus der Figur ist ersichtlich, daß die Immunglobulinsynthese durch Ba-Konzentration von 0,18 uM im Vergleich zur Kontrolle ohne Ba-Zusatz bereits deutlich inhibiert wird. Die angegebenen Prozentsätze beziehen sich dabei jeweils auf Kontroll-Zellkulturen ohne Ba-Zusatz.

### 2.2 Einfluß von Ba auf die IgG- und IgM-Synthese in vitro.

Der Versuch wurde mit Blutleukozyten von zwei gesunden Spendern wie unter 2.1 durchgeführt, mit dem Unterschied, daß statt des zuvor verwendeten monoklonalen Antikörpers, der mit allen menschlichen Immunglobulin-Spezies reagiert, für diesen Versuch spezifisch mit Immunglobulin G bzw. Immunglobulin M reagierende monoklonale Antikörper verwendet wurden. Für den Nachweis von IgG wurde dabei als erster Antikörper ein monoklonaler Antikörper gegen humane Gammaketten (Nr. 1410-KG7 von der ATCC, Bethesda, Maryland, USA; 200 ng/Napf) in die Mikrotiterplatten geschichtet wurde. Für den Nachweis von IgM wurde anstelle dessen ein monoklonaler Antikörper gegen humane u-Ketten (DA 4.4 von der ATCC, Bethesda, Maryland, U.S.A., 500 ng/Napf) in die Mikrotiterplatten geschichtet. Nach Inkubation mit jeweils 0,1 ml des Zellkultur-Überstandes wurde für den Nachweis beider Immunglobuline mit biotinylierten Ziegen-Immunglobulin (IgG) gegen IgG, IgM und IgA sowie Kappa- und Lambda-Ketten humaner Immunglobuline (Dianova, Hamburg) inkubiert. Der Nachweis des gebundenen biotinylierten Antikörpers wurde in beiden Fällen mit 1:1000 verdünntem Streptavidin-Peroxidasekonjugat (Amersham, Braunschweig) geführt.

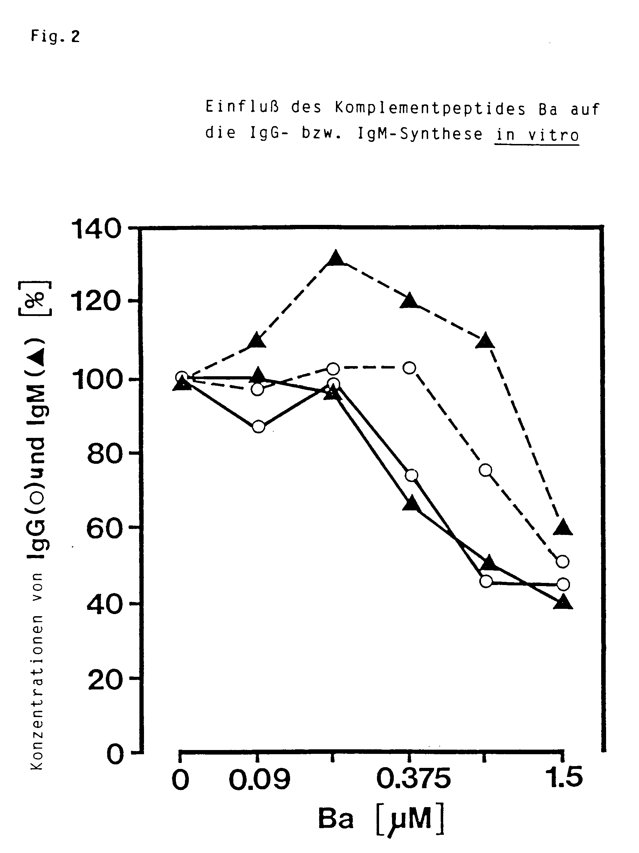

Die Ergebnisse dieses Versuches sind in Figur 2 gezeigt. Aus der Figur ist ersichtlich, daß die Zellkulturen bei Ba-Konzentrationen von über 0,375 uM eine stark verringerte IgG-Konzentration aufweisen, während die IgM-Konzentration in einem der beiden Fälle erst durch Ba-Konzentrationen von 1,5 uM beeinflußt wurde.

### 2.3 Einfluß von Ba auf die IgG-Synthese LPS-stimulierter Zellkulturen

Lipopolysaccharide (LPS) sind Endotoxine aus der Zellwand gramnegativer Bakterien, die als typische Aktivatoren von B-Lymphozyten gelten und deren Zusatz zu Zellkulturen zur Induktion einer Immunglobulinsynthese, insbesondere der IgG-Synthese führt. Im vorliegenden Versuch wurde der Einfluß von Ba auf die IgG- und IgM-Synthese nach Stimulation der Immunglobulinsynthese in Zellkulturen mononukleärer Blutzellen eines gesunden Spenders mit Lipopolysacchariden aus Salmonella minnesota (Sigma, Deisenhofen) gemessen. Die Konzentration der Zellen in der Zellkultur betrug wiederum 1,5 x 10⁶ mononukleäre Blutleukozyten/ml Serum-freies Iscove's Medium. Je 1 ml dieser Zellkulturen wurde wie oben 7 Tage bei 37°C (i) ohne Zusatz, (ii) unter Zusatz von 0,4 uM Ba, (iii) unter Zusatz von 8 ug/ml LPS (S. minnesota Re595) und (iv) 0,4 uM Ba + 8 ug/ml LPS (S. minnesota Re595) inkubiert. Anschließend wurde die IgG- bzw. IgM-Konzentration wiederum unter Verwendung eines ELISA-Tests mit den bereits in Beispiel 2.2 verwendeten Antikörpern bestimmt.

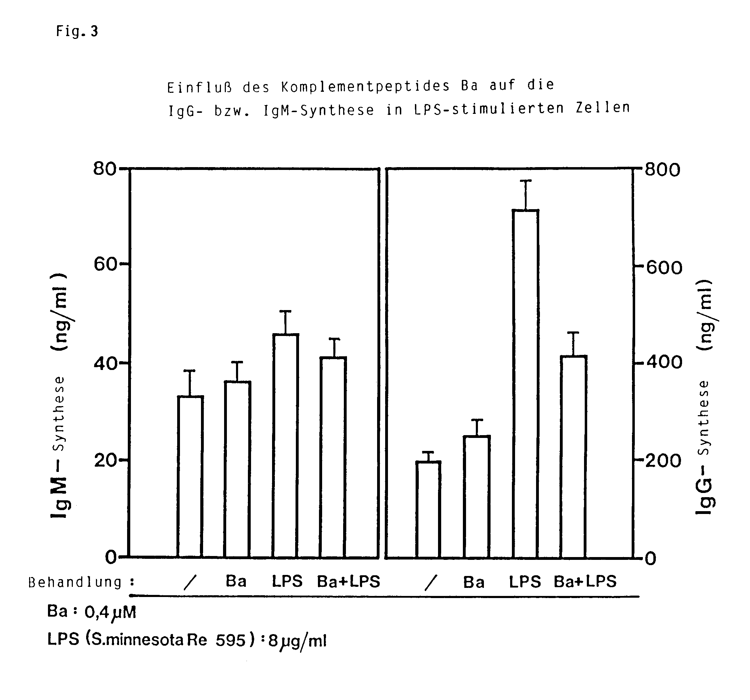

Die Ergebnisse dieses Versuches sind in Figur 3 gezeigt. Offensichtlich wird durch Inkubation LPS-stimulierter Zellen mit Ba bereits bei einer Ba-Konzentration von 0,4 uM die Stimulation der IgG-Synthese wirksam reduziert. Der Einfluß auf die IgM-Synthese war bei der verwendeten Ba-Konzentration weit weniger drastisch (vergleiche auch Figur 2).

### Beispiel 3

### Inhibition der Zellproliferation durch das Komplementpeptid Ba

Mit diesem Versuch soll gezeigt werden, daß nicht nur die Freisetzung von Immunglobulinen durch das Komplementpeptid Ba inhibiert wird, sondern auch die Zellproliferation mononukleärer Blutleukozyten, d.h. deren DNA-Synthese, unterdrückt wird. Eine Zellproliferation ist eine Vorbedingung für differenzierte Zellfunktionen, d.h. also auch für die Antikörper-Synthese. Insofern würde der Nachweis der Inhibition der Zellproliferation durch das Komplementpeptid Ba die Ergebnisse über die Inhibition der Antikörper-Synthese ergänzen.

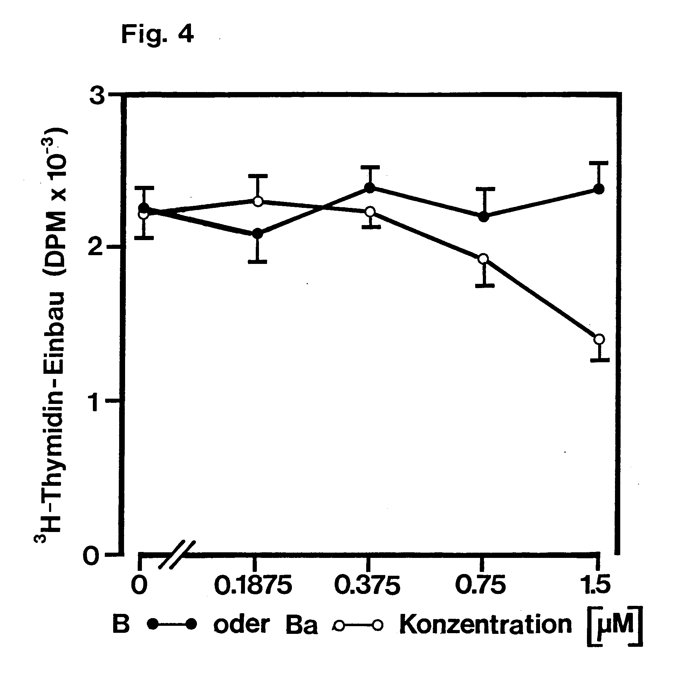

Für die Durchführung des Versuches wurden mononukleäre Blutleukozyten in einer Konzentration von 1,5 x 10⁶ Zellen/ml Iscove's Medium verwendet. Jeweils 0,1 ml Zellsuspension wurden mit Komplementprotein B bzw. Komplementpeptid Ba (Isolierung s. 2.1) in Konzentrationen zwischen 0 und 1,5 uM (s. Fig. 4) unter ansonsten den gleichen Bedingungen, wie in Beispiel 2, insgesamt 4 Tage kultiviert. Am 3. Tage der Kultur wurden pro Napf 0,25 uCi radioaktiv markiertes Thymidin (³H-Thymidin, 2 Ci/mMol, Amersham, Braunschweig) zugesetzt. Nach weiteren 24 Stunden Kultur wurden die Zellen durch einmaliges Einfrieren und Auftauen lysiert. Die freigesetzte makromolekulare DNA wurde auf Glasfaserfiltern in einem Zellsammler PHD der Fa. Cambridge Technology, Boston, U.S.A. durch Absaugen aus den Mikrotiterplatten gesammelt. Bei dieser Methode der Gewinnung makromolekularer DNA wird das nichteingebaute ³H-Thymidin auf die Filter gesaugt, die Filter werden gewaschen, die adhärente makromolekulare DNA (zusammen mit anderen makromolekularen Zellbestandteilen) bleibt auf dem Filter (Standardmethoden). Die Filterscheiben wurden bei 60°C im Trockenschrank getrocknet und dann im Beta-Szintillationszähler auf ³H analysiert.

Aus dem Vergleich des nach dem Zusatz von ³H-Thymidin in die makromolekulare DNA eingebauten radioaktiven Thymidins der Versuchszellen mit dem von Kontrollzellen, die keiner Behandlung mit Komplementpeptid Ba unterworfen worden sind, kann der Einfluß des Ba-Zusatzes auch auf die Zellproliferation bestimmt werden.

Das Ergebnis des Versuches ist in Figur 4 dargestellt. Aus der Figur wird deutlich, daß das Komplementprotein B im gesamten untersuchten Konzentrationsbereich zwischen 0,18 und 1,5 uM keinen Einfluß auf die Zellproliferation hat, d.h., es werden stets ungefähr gleichbleibende Mengen tritiierten Thymidins eingebaut. Andererseits bewirkt die Zugabe von Komplementpeptid Ba in Konzentrationen von höher als 0,375 uM eine Abnahme der DNA-Synthese, die bei einer Konzentration von 1,5 uM mehr als 33 % beträgt.

Das in den Beispielen 2 und 3 verwendete Iscove's Medium wurde auf der Grundlage des von Boehringer Mannheim bezogenen MEM Iscove-Mediums durch Ergänzung mit folgenden Zusätzen hergestellt:
Glutamin (200 mM): 20 ml/l
Transferrin: 5 mg/l
Insulin: 5 mg/l
Humanes Serumalbumin (reinst, Behringwerke, Marburg): 2,5 g/l
Sojabohnenlipide: 20 ml/l
Antibiotisch-antikmykotische Lösung (GIBCO/BRL, Eggenstein; Zusammensetzung: 10000 E Penicillin, 10000 ug Streptomycin, 25 ug Fungizone (alle Angaben pro ml) in physiologischer Kochsalzlösung): 10 ml/l.

## Patentansprüche

1. Mittel mit immunsuppressiver Wirkung, **dadurch gekennzeichnet,** daß es Peptide mit der biologischen Funktion eines oder mehrerer der Komplementpeptide Ba und/oder C3a und/oder C3dg und oder C3d sowie übliche Zusatz-, Verdünnungs- und/oder Hilfsmittel enthält.

2. Mittel nach Anspruch 1, **dadurch gekennzeichnet,** daß die Peptide durch proteolytische Spaltung natürlicher Komplementfaktoren, durch partielle oder totale biologische und/oder chemische Synthese hergestellt worden sind.

3. Mittel nach mindestens einem der Ansprüche 1 bis 2, **dadurch gekennzeichnet,** daß es als wässrige Lösung, als Wasser-in-Öl-Emulsion oder in Liposomen inkorporiert vorliegt.

4. Mittel nach mindestens einem der Ansprüche 1 bis 3, **dadurch gekennzeichnet,** daß es weitere immunsuppressive Wirkstoffe enthält.

5. Antikörper gegen den Komplementrezeptor Typ 2 (CR2), der die Bindung der Komplementpeptide C3dg/C3d blockiert, **dadurch gekennzeichnet,** daß er die Aggregation von Rezeptormolekülen inhibiert.

6. Antikörper nach Anspruch 5, **dadurch gekennzeichnet,** daß es ein monoklonaler Antikörper ist.

7. Monovalenter Antikörper, **dadurch gekennzeichnet,** daß er die Bindung der Komplementpeptide C3dg/C3d blockiert und die Aggregation von Rezeptormolekülen inhibiert.

8. Monovalenter Antikörper nach Anspruch 7, **dadurch gekennzeichnet,** daß er ein Fragment eines Antikörpers nach Anspruch 5 und/oder 6 ist.

9. Antikörpers nach mindestens einem der Ansprüche 5 bis 8 als Anzheimittel zur Inhibition der Aggregation des Komplementrezeptors Typ 2 (CR2).

10. Zusammensetzung, **dadurch gekennzeichnet,** daß sie einen Antikörper nach Anspruch 5 und/oder 6 und/oder einen monovalenten Antikörper nach Anspruch 7 und/oder 8 enthält.

11. Zusammensetzung nach Anspruch 10, **dadurch gekennzeichnet**, daß sie weitere immunsuppressive Wirkstoffe enthält.

12. Zusammensetzung nach Anspruch 10 und/oder 11, **dadurch gekennzeichnet,** daß sie übliche Zusatz-, Verdünnungs- und/oder Hilfsmittel enthält.

13. Mittel nach mindestens einem der Ansprüche 1 bis 4, **dadurch gekennzeichnet,** daß es den Antikörper nach mindestens einem der Ansprüche 5 bis 8 enthält.

## Claims

1. Preparation having immunosuppressive action, characterised in that it contains peptides having the biological function of one or more of the complement peptides Ba and/or C3a and/or C3dg and/or C3d, and customary additives, diluents and/or auxiliaries.

2. Preparation according to Claim 1, characterised in that the peptides have been prepared by proteolytic cleavage of natural complement factors, or by partial or total biological and/or chemical synthesis.

3. Preparation according to at least one of Claims 1 or 2, characterised in that it is present as an aqueous solution, as a water-in-oil emulsion or incorporated in liposomes.

4. Preparation according to at least one of Claims 1 to 3, characterised in that it contains further immunosuppressive active ingredients.

5. Antibody against the complement receptor type 2 (CR2) which blocks the binding of the complement peptides C3dg/C3d, characterised in that it inhibits the aggregation of receptor molecules.

6. Antibody according to Claim 5, characterised in that it is a monoclonal antibody.

7. Monovalent antibody, characterised in that it blocks the binding of the complement peptides C3dg/C3d and inhibits the aggregation of receptor molecules.

8. Monovalent antibody according to Claim 7, characterised in that it is a fragment of an antibody according to Claim 5 and/or 6.

9. Antibody according to at least one of Claims 5 to 8 as a medicament for inhibiting the aggregation of the complement receptor type 2 (CR2).

10. Composition, characterized in that it contains an antibody according to Claim 5 and/or 6, and/or a monovalent antibody according to Claim 7 and/or 8.

11. Composition according to Claim 10, characterized in that it contains further immunosuppressive active ingredients.

12. Composition according to Claim 10 and/or 11, characterized in that it contains customary additives, diluents and/or auxiliaries.

13. Preparation according to at least one of Claims 1 to 4, characterized in that it contains the antibody according to at least one of Claims 5 to 8.

## Revendications

1. Agent possédant un effet immuno-suppresseur, caractérisé par le fait qu'il contient des peptides présentant la fonction biologique d'un ou plusieurs des peptides complémentaires Ba et/ou C3a et/ou C3dg et/ou C3d, ainsi que les additifs diluants et/ou auxiliaires habituels.

2. Agent selon la revendication 1, caractérisé par le fait que l'on a fabriqué les peptides par dissociation proté olytique de facteurs complémentaires naturels, par synthèse biologique et/ou chimique, partielle ou totale.

3. Agent selon au moins l'une des revendications 1 à 2, caractérisé par le fait qu'il se présente sous forme de solution aqueuse, sous forme d'émulsion eau dans huile ou incorporé dans des liposomes.

4. Agent selon au moins l'une des revendications 1 à 3, caractérisé par le fait qu'il contient d'autres principes actifs immuno-suppresseurs.

5. Anticorps à l'égard du récepteur complémentaire type 2 (CR2), qui bloque la liaison des peptides complémentaires C3dg/C3d, caractérisé par le fait qu'il intervient comme inhibiteur de l'agrégation des molécules du récepteur.

6. Anticorps selon la revendication 5, caractérisé par le fait qu'il est un anticorps monoclonal.

7. Anticorps monovalent, caractérisé par le fait qu'il bloque la liaison des peptides complémentaires C3dg/C3d et qu'il intervient comme inhibiteur de l'agrégation des molécules du récepteur.

8. Anticorps monovalent selon la revendication 7, caractérisé par le fait qu'il est un fragment d'un anticorps selon la revendication 5 et/ou 6.

9. Anticorps selon au moins l'une des revendications 5 à 8, utilisé comme remède pour inhiber l'agrégation du récepteur complémentaire, type 2 (CR2).

10. Synthèse caractérisée par le fait qu'elle contient un anticorps selon la revendication 5 et/ou 6 et/ou un anticorps monovalent selon la revendication 7 et/ou 8.

11. Synthèse selon la revendication 10, caractérisée par le fait qu'elle contient d'autres principes actifs immuno-suppresseurs.

12. Synthèse selon la revendication 10 et/ou 11, caractérisée par le fait qu'elle contient des additifs, des diluants et/ou des auxiliaires habituels.

13. Agent selon au moins l'une des revendications 1 à 4, caractérisé par le fait qu'il contient l'anticorps selon au moins l'une des revendications 5 à 8.
